**Europäisches Patentamt**

**European Patent Office**

**Office européen des brevets**

(19)

(11) Veröffentlichungsnummer : **0 475 286 B1**

(12) ## EUROPÄISCHE PATENTSCHRIFT

(45) Veröffentlichungstag der Patentschrift :
**05.01.94 Patentblatt 94/01**

(51) Int. Cl.$^5$ : **A61K 6/04,** C22C 19/07,
A61C 13/00

(21) Anmeldenummer : **91115046.4**

(22) Anmeldetag : **06.09.91**

(54) **Metallegierung für gegossene Prothesengerüste in der Zahnheilkunde.**

(30) Priorität : **12.09.90 DE 4028870**

(43) Veröffentlichungstag der Anmeldung :
**18.03.92 Patentblatt 92/12**

(45) Bekanntmachung des Hinweises auf die
Patenterteilung :
**05.01.94 Patentblatt 94/01**

(84) Benannte Vertragsstaaten :
**AT DE FR GB IT**

(56) Entgegenhaltungen :
**EP-A- 0 080 074
EP-A- 0 164 329
AT-B- 370 616**

(56) Entgegenhaltungen :
**DE-A- 3 609 184
DE-A- 3 941 820
DE-C- 2 225 577
DE-C- 3 415 249
DE-C- 3 436 118
DE-C- 3 510 331
DE-C- 3 744 491
US-A- 4 514 359
US-A- 4 530 664**

(73) Patentinhaber : **Thyssen Edelstahlwerke AG
August-Thyssen-Strasse 1
D-40211 Düsseldorf (DE)**

(72) Erfinder : **Weigand, Hans-Hermann, Dr.-Ing.
Brucknerstrasse 5
W-4154 Tönisvorst 2 (DE)**

**Beschreibung**

Für die Herstellung von Prothesengerüsten für herausnehmbaren Zahnersatz werden bislang vorwiegend kohlenstoffhaltige Kobalt-Chrom-Molybdän-Legierungen, auch Modellgußlegierungen genannt, verwendet. Bekannte Legierungen dieses Typs enthalten 0,3 bis 0,6 % C, jeweils bis 1 % Si und Mn, 27 bis 32 % Cr, 3 bis 8 % Mo, Rest Kobalt mit den üblichen herstellungsbedingten Verunreinigungen ("Das Dental Vademecum", Verzeichnis zahnärztlicher und zahntechnischer Arbeitsmittel und Werkstoffe. Herausgeber Bundesärztekammer (Bundesverband der deutschen Zahnärtze, BDZ); Ausgabe 1989/90). Der Molybdängehalt dieser Legierungen kann nach DIN 13912 Teil 2 (Entwurf) ganz oder teilweise durch Wolfram ersetzt werden, wobei Wolfram bezüglich seines Einflusses auf die Korrosionsbeständigkeit etwa halb so wirksam wie Molybdän ist. Diese Legierungen besitzen im allgemeinen für den vorgesehenen Verwendungszweck befriedigende mechanische und korrosionschemische Eigenschaften. Ihre verhältnismäßig geringe Verformbarkeit mit Werten für die Bruchdehnung ($A_5$) zwischen 2 und 6 % läßt jedoch nur geringe plastische Verformungen ohne Bruchgefahr zu.

Zur Verbesserung der Verformbarkeit, die zum Beispiel bei Richtarbeiten an den Halteklammern von Prothesengerüsten erforderlich ist, sind titanhaltige Legierungen und außerdem Werkstoffe vorgeschlagen worden, bei denen ein Teil des Kohlenstoffs durch Stickstoff ersetzt wurde (DE 2 225 577 C2). Diese Legierungen besitzen verhältnismäßig hohe Werte für die 0,2 %-Dehngrenze (rd. 650 N/mm$^2$) und Bruchdehnungen bis zu 12 %. Eine zur Herstellung leichterer und grazilerer Prothesengerüste durchaus wünschenswerte Anhebung der 0,2 %-Dehngrenze auf Werte oberhalb 670 N/mm$^2$ ist jedoch durch Stickstoffzusatz bei der gegebenen Legierungsbasis nicht möglich, weil bei Stickstoffgehalten oberhalb 0,3 % die Löslichkeitsgrenze erreicht wird, was die Herstellung porenfreier Gußobjekte unmöglich macht.

Der Erfindung liegt die Aufgabe zugrunde, eine korrosionsbeständige und unter den Bedingungen eines Dentallabors gut vergießbare Legierung zu schaffen, die bei hoher mechanischer Belastbarkeit (0,2 %-Dehngrenze oberhalb 670 N/mm$^2$) und ausreichend hohe Bruchverformungswerte (Bruchdehnung $A_5 \geqq$ 8 %) aufweist. Diese Aufgabe wird erfindungsgemäß durch eine Metallegierung gelöst, die folgende Zusammensetzung in Masse-% hat:

```
 0,1 bis 0,3  % C
     bis 1,5  % Si
     bis 3    % Mn
25   bis 35   % Cr
 3   bis 6,5  % Mo
 0,5 bis 5    % Ta
 0,15 bis 0,40 % N
Rest Kobalt einschließlich erschmelzungsbedingter
Verunreinigungen.
```

Bevorzugt übersteigt der Molybdängehalt den Tantalgehalt und der Summengehalt (%Mo) + (%Ta) ist größer/gleich 4,5 %.

Die Kobaltlegierung kann ferner bis 0,5 % Bor, vorzugsweise 0,01 bis 0,1 % Bor, enthalten. Bor verbessert in diesem Bereich die Dünnflüssigkeit und die Zähigkeit der Legierung.

Überraschenderweise wurde nämlich festgestellt, daß ein Zusatz von Tantal zu einer bemerkenswerten Steigerung der mechanischen Eigenschaften führt, ohne daß die übrigen geforderten Eigenschaften, wie Korrosionsbeständigkeit oder Verarbeitbarkeit, nennenswert beeinträchtigt werden. Dies soll anhand einiger Beispiele nachfolgend erläutert werden.

Beispiel 1:

Die nachstehende Tabelle 1 gibt eine Darstellung der an gegossenen Zugproben ermittelten mechanischen Eigenschaften.

## Tabelle 1

| Legie-rung Nr. | C | Cr | Mo | Ta (in %) | N | Sonstige | 0,2-Grenze $R_{p0,2}$ $N/mm^2$ | Zugfestig-keit $R_m$ $N/mm^2$ | Bruch-dehnung $A_5$ in % | Bruch-einschn. Z in % |
|---|---|---|---|---|---|---|---|---|---|---|
| 1 | 0,2 | 29 | 4,93 | - | 0,26 | Si, B | 638 | 924 | 12,5 | 20,3 |
| 2 | 0,17 | 29,8 | 4,14 | - | 0,30 | Si, B | 665 | 947 | 11,7 | 17,7 |
| 3 | 0,21 | 29,2 | 3,1 | 1,07 | 0,23 | Si, B | 591 | 875 | 11,7 | 13,7 |
| 4 | 0,17 | 29,0 | 0,05 | 5,26 | 0,29 | Si, B | 468 | 799 | 8,8 | 10,0 |
| 5 | 0,19 | 29,0 | 2,94 | 1,92 | 0,21 | Si, B | 686 | 1057 | 13,2 | 16,0 |
| 6 | 0,21 | 28,6 | 4,94 | 2,08 | 0,27 | Si, B | 718 | 1015 | 8,0 | 11,0 |

Die Legierungen 1 und 2 entsprechen denen nach DE 2 225 577 C2.

Die Legierungen 3 und 4 sind Vergleichslegierungen außerhalb des beanspruchten Bereichs.

Die Legierungen 5 und 6 haben erfindungsgemäße Zusammensetzung.

Die zum Vergleich aufgeführten Legierungen 1 und 2, die in ihrer Zusammensetzung den Ansprüchen nach DE 2 225 577 C2 entsprechen, erreichen verhältnismäßig günstige Festigkeits- und Bruchverformungskennwerte. Wie bereits erwähnt, ist jedoch auf dieser Legierungsbasis eine weitere Steigerung der mechanischen Eigenschaften nicht möglich. Die Legierungen mit Tantalzusatz führen nicht in jedem Falle zu der gewünschten Eigenschaftsverbesserung, wie die Werte der nicht erfindungsgemäßen Vergleichslegierungen 3 und 4 zeigen. Erst die erfindungsgemäße Abstimmung der Molybdän- und Tantalgehalte bei den Legierungen 5 und 6 ergibt bemerkenswert günstige Werte für die 0,2 %-Dehngrenze auf über 680 N/mm² und Bruchdehnungswerte von 8 % und höher.

Beispiel 2:

An bekannten und erfindungsgemäßen Kobaltlegierungen wurden zur Prüfung der Korrosionsbeständigkeit Stromdichte - Potential - Kurven in künstlichem Speichel nach Fusayama mit und ohne Spalt aufgenommen. Als Kriterium für die Korrosionsbeständigkeit diente einmal das Durchbruchpotential und bei den Proben mit Spalt (aufgeschobener Kunststoffring) auch das Repassivierungspotential. Die gemessenen Werte sind in der folgenden Tabelle 2 zusammengestellt.

## Tabelle 2

| Legie-rung Nr. | C | Cr | Mo | Ta (in %) | N | Sonstige | Durchbruch-potential in mV a) | Durchbruch-potential in mV b) | Repassivierungs-potential in mV b) |
|---|---|---|---|---|---|---|---|---|---|
| 1 | 0,2 | 29 | 4,93 | - | 0,26 | Si, B | +750 | +780 | +550 |
| 7 | 0,2 | 28,6 | 4,97 | 1,50 | 0,26 | Si, B | +610 | +640 | +640 |

1 - nach DE 2 225 577 C2        a) ohne Spalt
7 - erfindungsgemäß                b) mit Spalt

Aus den Ergebnissen in Tabelle 2 geht hervor, daß alle Kobaltlegierungen infolge ihrer Durchbruchpotentiale oberhalb + 600 mV als mundbeständig anzusehen sind, da im Munde Potentiale von höchstens + 300 mV auftreten. Die Repassivierungspotentiale liegen bei der erfindungsgemäßen Legierung bei oder oberhalb der Durchbruchpotentiale, so daß die Legierungen auch unter Spaltbedingungen leicht repassivieren, was ihre Eignung für eine Verwendung im Munde unterstreicht.

**Patentansprüche**

1.   Metallegierung für gegossene Prothesengerüste in der Zahnheilkunde, bestehend aus (in Masse-%)

```
0,1 bis 0,3  % C
     bis 1,5  % Si
     bis 3    % Mn
25   bis 35   % Cr
 3   bis 6,5  % Mo
 0,5 bis 5    % Ta
0,15 bis 0,40 % N
Rest Kobalt einschließlich erschmelzungsbedingter
Verunreinigungen.
```

2.   Legierung der Zusammensetzung nach Anspruch 1, bei der der Molybdängehalt den Tantalgehalt übersteigt und der Summengehalt (% Mo) + (% Ta) größer/gleich 4,5 % ist.

3.   Legierung der Zusammensetzung nach Anspruch 1 oder 2, die zusätzlich bis 0,5 % B, vorzugsweise 0,01 bis 0,1 % B, enthält.

**Claims**

1.   A metal alloy for moulded prosthetic constructions in dentistry, comprising (in % by weight)

```
0.1  to   0.3 % C
     up to 1.5 % Si
     up to 3   % Mn
25        to 35  % Cr
 3        to 6.5 % Mo
 0.5 to   5   % Ta
0.15 to   0.40% N
```

residue cobalt, including impurities due to melting.

2.   An alloy of the composition according to claim 1, wherein the molybdenum content exceeds the tantalum content and the total content (% Mo) + (% Ta) is greater than/equal to 4.5%.

3.   An alloy of the composition according to claims 1 or 2, which also contains up to 0.5% B, preferably 0.01 to 0.1% B.

**Revendications**

1.   Alliages métalliques pour armatures de-prothèse dentaires moulées comprenant ( en % massiques):
entre 0.1 et 0.3 % C
jusqu'à 1.5 % Si
jusqu'à 3 % Mn

entre 25 et 35 % Cr
entre 3 et 6.5 % Mo
entre 0.5 et 5 % Ta
entre 0.15 et 0.40 % N
le reste étant du cobalt, en comprenant les impuretés résultant de la fusion.

2. Alliage ayant une composition selon la revendication 1, dans lequel la teneur en molybdène dépasse la teneur en tantale et la somme des teneurs (% Mo)+(% Ta) est supérieure ou égale à 4.5 %.

3. Alliage ayant une composition selon la revendication 1 ou 2, et qui contient de plus jusqu'à 0.5 % B, de préférence entre 0.01 et 0.1 % B.